# EUROPÄISCHE PATENTANMELDUNG

(11) **EP 3 459 960 A1**
(43) Veröffentlichungstag der Anmeldung: **27.03.2019**
(21) Anmeldenummer: 17193150.4
(22) Anmeldetag: 26.09.2017
(51) Int. Cl.: C07F 9/6574, B01J 31/18, C07C 45/50

(54) **BISPHOSPHITLIGANDEN MIT DIOXAPHOSPHEPIN-FLÜGELBAUSTEINEN**

(71) Anmelder: Evonik Degussa GmbH, 45128 Essen (DE)
(72) Erfinder: ZHANG, Baoxin, 18106 Rostock (DE); BÖRNER, Armin, 18059 Rostock (DE); FRANKE, Robert, 45772 Marl (DE)

(57) **Zusammenfassung**

Bisphosphitliganden mit Dioxaphosphepin-Flügelbausteinen und deren Verwendung als Liganden in der Hydroformylierung.

## Beschreibung

Die Erfindung betrifft Bisphosphitliganden mit Dioxaphosphepin-Flügelbausteinen und deren Verwendung als Liganden in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

Der Erfindung lag die Aufgabe zugrunde, Bisphosphitliganden bereitzustellen, welche als Liganden in der Hydroformylierung eingesetzt werden können, und dort eine sehr gute Gesamtausbeute an Aldehyd liefern.

Gelöst wird die Aufgabe durch eine Verbindung gemäß Anspruch 1.

Verbindung der Formel (**I**): 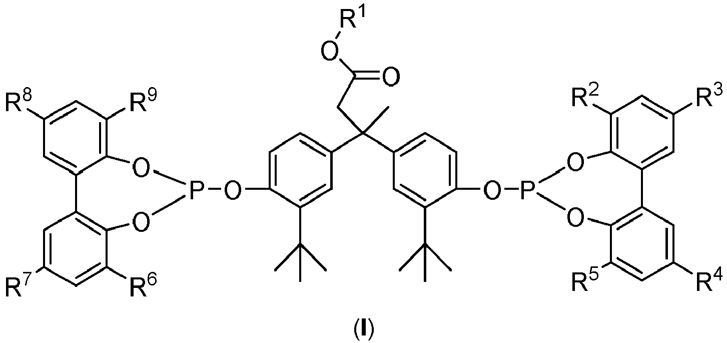 wobei
R¹ für -(C₁-C₁₂)-Alkyl steht;
R², R³, R⁴, R⁵, R⁶, R⁷, R⁸, R⁹ ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, Halogen,
wobei die genannten Alkylgruppen und Arylgruppen wie folgt substituiert sein können: -(C₁-C₁₂)-Alkyl, -O-(C₁-C₁₂)-Alkyl, Halogen;
und mindestens einer der Reste R², R³, R⁴, R⁵, R⁶, R⁷, R⁸, R⁹ nicht für -H steht.

In einer Ausführungsform steht R¹ für -CH₃.

In einer Ausführungsform stehen R², R⁵, R⁶, R⁹ für -(C₁-C₁₂)-Alkyl.

In einer Ausführungsform stehen R², R⁵, R⁶, R⁹ für *-tert-*Bu*.*

In einer Ausführungsform sind R³, R⁴, R⁷, R⁸ ausgewählt aus: -(C₁-C₁₂)-Alkyl, -O-(C₁-C₁₂)-Alkyl.

In einer Ausführungsform stehen R³, R⁴, R⁷, R⁸ für -(C₁-C₁₂)-Alkyl.

In einer Ausführungsform stehen R³, R⁴, R⁷, R⁸ für *-tert-*Bu*.*

In einer Ausführungsform stehen R³, R⁴, R⁷, R⁸ für -O-(C₁-C₁₂)-Alkyl.

In einer Ausführungsform stehen R³, R⁴, R⁷, R⁸ für -O-CH₃.

In einer Ausführungsform weist die Verbindung die Struktur (**1**) auf:

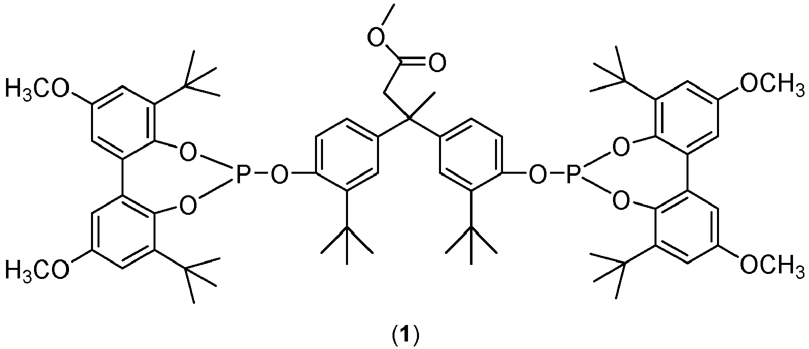

In einer Ausführungsform weist die Verbindung die Struktur (**2**) auf:

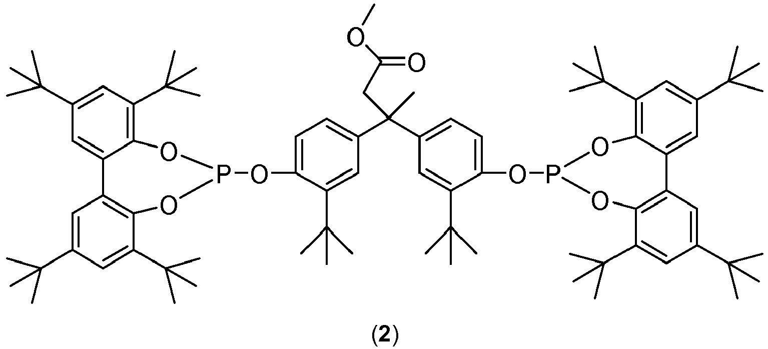

Neben der Verbindung als Stoff wird auch dessen Verwendung als Ligand in einer Hydroformylierungsreaktion beansprucht.

Verwendung einer zuvor beschriebenen Verbindung in einem Ligand-Metall-Komplex zur Katalyse einer Hydroformylierungsreaktion.

Des Weiteren wird auch ein Verfahren beansprucht, in welchem eine zuvor beschriebene Verbindung eingesetzt wird.

Verfahren umfassend die Verfahrensschritte:
a) Vorlegen eines Olefins,
b) Zugabe eines Komplexes, welcher eine zuvor beschriebene Verbindung umfasst, sowie ein Metall ausgewählt aus: Rh, Ru, Co, Ir,
   oder einer zuvor beschriebene Verbindung und einer Substanz, welche ein Metall ausgewählt aus: Rh, Ru, Co, Ir aufweist;
c) Zuführen von H₂ und CO,
d) Erwärmen des Reaktionsgemisches, wobei das Olefin zu einem Aldehyd umgesetzt wird.

In einer Variante des Verfahrens ist das Metall Rh.

Im Folgenden wird die Erfindung anhand von Ausführungsbeispielen näher erläutert.

### Ligandensynthese

### Ligand (1)

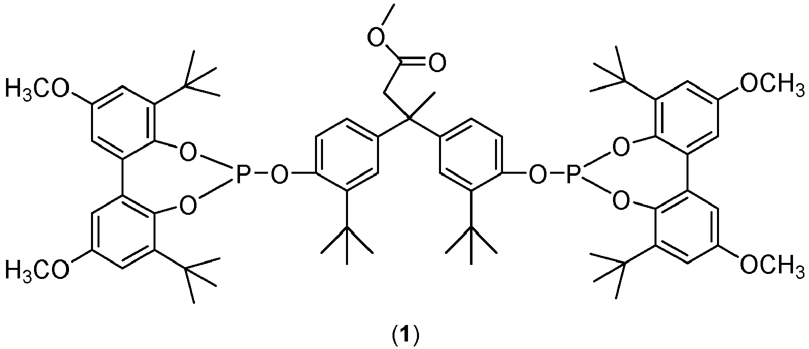

Eine gerührte Lösung von 4,8-Di-*tert*-butyl-6-chlor-2,1 0-dimethoxydibenzo[*d,f*][1,3,2]-dioxaphosphepin (2,28 g; 5,39 mmol) in THF (12 ml) wird bei 0 °C tropfenweise mit einer Mischung aus Methyl 3,3-bis(3-(*tert*-butyl)-4-hydroxyphenyl)butanoate (0,967 g; 2,43 mmol), Triethylamin (1,5 ml) und THF (12 ml) versetzt. Man lässt über Nacht bei Raumtemperatur rühren, filtriert, engt das Filtrat im Vakuum ein und trocknet den erhaltenen Rückstand 2 h bei 50°C / 0,1 mbar. Die säulenchromatografische Reinigung (n-Heptan/Dichlormethan = 1/2, R_{f} = 0,11) ergibt 0,81 g (0,691 mmol; 28,5%) des Biphosphites.
Analyse: ³¹P-NMR (CD₂Cl₂): 139,3 (s) ppm.
¹H-NMR (CD₂Cl₂): 1,20 (18H); 1,37 (36H); 1,84 (3H); 3,06 (2H); 3,41 (3H); 3,82 (12H); 6,77-7,10 (14H) ppm.
ESI-TOF HRMS: *m*/*e* 1171,58267 (M+H)⁺.

### Ligand (2)

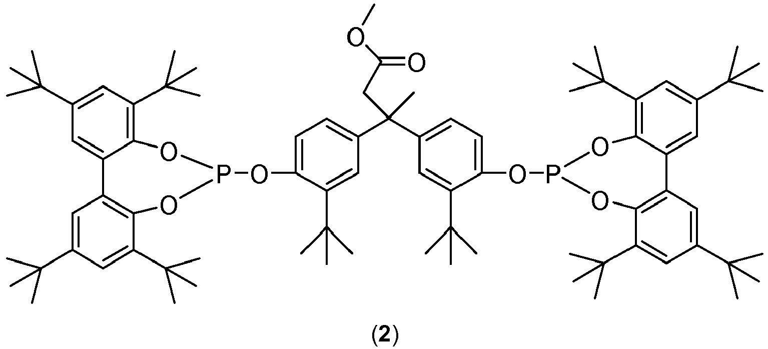

Eine gerührte Lösung von 2,4,8,10-Tetra-*tert*-butyl-6-chlordibenzo[*d,f*[1,3,2]-dioxaphosphepin (2,08 g; 4,39 mmol) in THF (12 ml) wird bei 0 °C tropfenweise mit einer Mischung aus Methyl 3,3-bis(3-(*tert*-butyl)-4-hydroxyphenyl)butanoate (0,69 g; 1,73 mmol), Triethylamin (1,3 ml) und THF (12 ml) versetzt. Man lässt über Nacht bei Raumtemperatur rühren, filtriert, engt das Filtrat im Vakuum ein und trocknet den erhaltenen Rückstand 2 h bei 50°C / 0,1 mbar. Die säulenchromatografische Reinigung (n-Heptan/Dichlormethan = 2/1, R_{f} = 0,27) ergibt 1,45 g (1,14 mmol; 65,7%) des Biphosphites.
Analyse: ³¹P-NMR (CD₂Cl₂): 140,8 (s) ppm.
¹H-NMR (CD₂Cl₂): 1,16 (18H); 1,36 (36H); 1,39 (36H); 1,83 (3H); 3,04 (2H); 3,41 (3H); 6,93-7,06 (6H); 7,23 (d, 4H); 7,44 (d, 4H) ppm.
ESI-TOF HRMS: *m*/*e* 1275,79046 (M+H)⁺.

### Ligand (3) (Vergleichsligand)

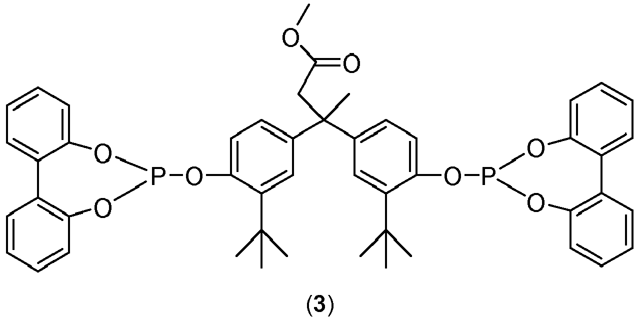

Eine gerührte Lösung von 6-Chlordibenzo[*d,f*][1,3,2]-dioxaphosphepin (7,08 g; 28,23 mmol) in THF (30 ml) wird bei 0 °C tropfenweise mit einer Mischung aus Methyl 3,3-bis(3-(*tert*-butyl)-4-hydroxyphenyl)butanoate (4,50 g; 11,29 mmol), Triethylamin (4,70 ml) und THF (30 ml) versetzt. Man lässt über Nacht bei Raumtemperatur rühren, filtriert, engt das Filtrat im Vakuum ein und trocknet den erhaltenen Rückstand 2 h bei 50°C / 0,1 mbar. Die säulenchromatografische Reinigung (n-Heptan/Dichlormethan = 1/1, R_{f} = 0,27) ergibt 3,55 g (4,29 mmol; 38,0%) des Biphosphites.
Analyse: ³¹P-NMR (CD₂Cl₂): 145,0 (s) ppm.
¹H-NMR (CD₂Cl₂): 1,33 (18H); 1,89 (3H); 3,12 (2H); 3,46 (3H); 7,04...7,55 (22H) ppm.
ESI-TOF HRMS: *m*/*e* 827,28918 (M+H)⁺.

### Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt. Das als Substrat eingesetzte Gemisch der n-Octene (Oxeno GmbH, Octenisomerengemisch aus 1-Octen: 3 %; *cis*+*trans-*2*-*Octen*:* 49 %; *cis*+*trans-*3*-*Octen: 29 %; *cis*+*trans-*Octen*-*4: 16 %; gerüstisomere Octene: 3 %) wurden über Natrium am Rückfluß erhitzt und unter Argon destilliert.

Im Autoklaven wurden unter Argonatmosphäre Lösungen der Katalysatorvorstufe und des Liganden gemischt. Als Katalysatorvorstufe kam [(acac)Rh(COD)] (Umicore, acac = Acetylacetonat-Anion; COD = 1,5-Cyclooctadien) zum Einsatz. Von der Rhodiumverbindung wurden 10 ml einer 4,31 millimolaren Lösung in den Autoklaven gegeben. Anschließend wurde die entsprechende Menge an Liganden (P/Rh = 4:1) in 10 ml Toluol gelöst und zugemischt. Durch Zugabe von weiterem Toluol wurde das Anfangsvolumen der Katalysatorlösung auf 41,0 ml eingestellt. In eine druckfeste Pipette wurden die n-Octene (10,70 g; 95,35 mmol) eingefüllt. Der Autoklav wurde bei einem Gesamtgasdruck (Synthesegas: Linde; H₂ (99,999%): CO (99,997%) = 1:1) von 42 bar aufgeheizt. Nach Erreichen der Reaktionstemperatur von 120 °C wurde der Synthesegasdruck auf 48,5 bar erhöht und das Olefin mit einem in der Druckpipette eingestellten Überdruck von ca. 3 bar in den Autoklaven gepresst. Die Reaktion wurde bei konstantem Druck von 50 bar (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 10 ml Pentan verdünnt und gaschromatographisch analysiert: HP 5890 Series II plus, PONA, 50 m x 0,2 mm x 0,5 µm.

### Ergebnisse der Katalyseversuche

**Tabelle: Hydroformylierung der n-Octene**

| Ligand | Gesamtausbeute Aldehyd (%) |
|---|---|
| **1*** | 97,0 |
| **2*** | 98,3 |
| **3** | 65,7 |

| | |
|---|---|
| * erfindungsgemäße Verbindung | |

Wie die oben beschriebenen Versuche zeigen, wird die Aufgabe durch eine erfindungsgemäße Verbindung gelöst.

## Patentansprüche

1. Verbindung der Formel (**I**): 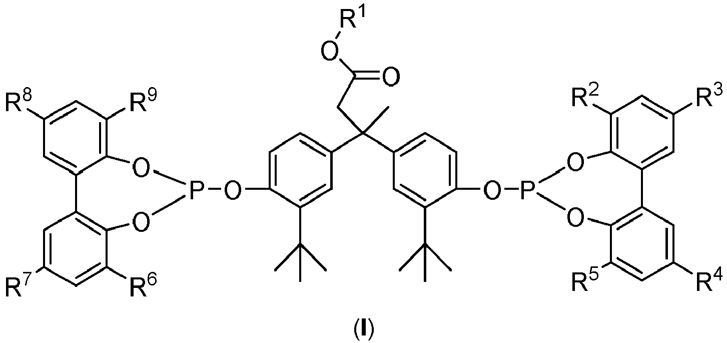 wobei
R¹ für -(C₁-C₁₂)-Alkyl steht;
R², R³, R⁴, R⁵, R⁶, R⁷, R⁸, R⁹ ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, Halogen,
wobei die genannten Alkylgruppen und Arylgruppen wie folgt substituiert sein können: -(C₁-C₁₂)-Alkyl, -O-(C₁-C₁₂)-Alkyl, Halogen;
und mindestens einer der Reste R², R³, R⁴, R⁵, R⁶, R⁷, R⁸, R⁹ nicht für -H steht.

2. Verbindung nach Anspruch 1,
wobei R¹ für -CH₃ steht.

3. Verbindung nach einem der Ansprüche 1 oder 2,
wobei R², R⁵, R⁶, R⁹ für -(C₁-C₁₂)-Alkyl stehen.

4. Verbindung nach einem der Ansprüche 1 bis 3,
wobei R², R⁵, R⁶, R⁹ für -*tert*-Bu stehen.

5. Verbindung nach einem der Ansprüche 1 bis 4,
wobei R³, R⁴, R⁷, R⁸ ausgewählt sind aus: -(C₁-C₁₂)-Alkyl, -O-(C₁-C₁₂)-Alkyl.

6. Verbindung nach einem der Ansprüche 1 bis 5,
wobei R³, R⁴, R⁷, R⁸ für -(C₁-C₁₂)-Alkyl stehen.

7. Verbindung nach einem der Ansprüche 1 bis 6,
wobei R³, R⁴, R⁷, R⁸ für *-tert-*Bu stehen.

8. Verbindung nach einem der Ansprüche 1 bis 5,
wobei R³, R⁴, R⁷, R⁸ für -O-(C₁-C₁₂)-Alkyl stehen.

9. Verbindung nach einem der Ansprüche 1 bis 5,
wobei R³, R⁴, R⁷, R⁸ für -O-CH₃ stehen.

10. Verbindung nach einem der Ansprüche 1 bis 5,
welche die Struktur (**1**) aufweist:

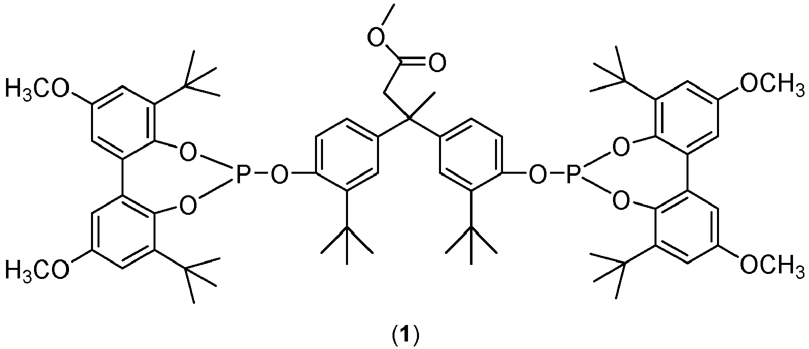

11. Verbindung nach einem der Ansprüche 1 bis 5,
welche die Struktur (**2**) aufweist:

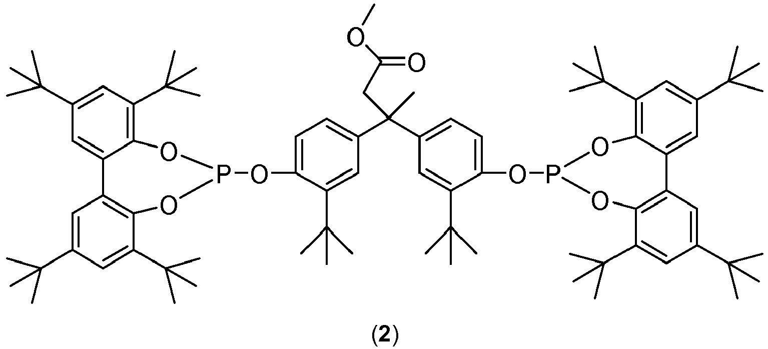

12. Verwendung einer Verbindung nach Anspruch 1 bis 11,
in einem Ligand-Metall-Komplex zur Katalyse einer Hydroformylierungsreaktion.

13. Verfahren umfassend die Verfahrensschritte:
a) Vorlegen eines Olefins,
b) Zugabe eines Komplexes, welcher eine Verbindung gemäß den Ansprüchen 1 bis 11 umfasst, sowie ein Metall ausgewählt aus: Rh, Ru, Co, Ir,
oder einer Verbindung nach einem der Ansprüche 1 bis 11 und einer Substanz, welche ein Metall ausgewählt aus: Rh, Ru, Co, Ir aufweist;
c) Zuführen von H₂ und CO,
d) Erwärmen des Reaktionsgemisches, wobei das Olefin zu einem Aldehyd umgesetzt wird.
